# EUROPEAN PATENT SPECIFICATION

(11) **EP 2 858 725 B1**
(45) Date of publication and mention of the grant of the patent: **29.08.2018**
(21) Application number: 13735055.9
(22) Date of filing: 14.05.2013
(51) Int. Cl.: A63B 21/00

(54) **RESPIRATORY THERAPY APPARATUS**
ATEMTHERAPIEVORRICHTUNG
APPAREIL DE THÉRAPIE RESPIRATOIRE

(30) Priority: 06.06.2012 GB 201209962
(43) Date of publication of application: 15.04.2015
(73) Proprietor: Smiths Medical International Limited, Ashford, Kent TN25 4BF (GB)
(72) Inventor: PETTITT, Emily, Bristol BS2 8BL (GB)
(74) Representative: Flint, Jonathan McNeill
(86) International application number: PCT/GB2013/000214
(87) International publication number: WO 2013/182833

(56) References cited:
- WO-A1-00/78407
- WO-A1-90/09203
- WO-A1-2010/000439
- US-A- 5 984 873
- US-A1- 2008 053 452

## Description

This invention relates to respiratory therapy apparatus of the kind including a mouthpiece, a first air opening, a restriction to flow of air between the mouthpiece and the opening against which the patient breathes and means to record use of the apparatus and provide an indication thereof.

The apparatus is more particularly concerned with apparatus for use in improving the performance of inspiratory muscles such as in patients suffering from COPD or similar respiratory problems, or for athletes and others seeking to reduce breathlessness.

Apparatus is presently available for training inspiratory muscles, incorporating some form of resistance against which the user inhales. By using such apparatus repeatedly over a prolonged period it is possible to improve lung function. Apparatus presently available includes the Powerbreathe and Powerbreathe Kinetic from Powerbreathe Medic, PowerLung from PowerLung Inc, Ultrabreathe from Tangent Healthcare Ltd, and Voldyne 5000 from Hudson RCI. Respiratory therapy apparatus is also described in US6553746, GB2278545A, GB2451593B, US6726598, US4854574 and US4533137. WO00/78407 describes a pulmonary exercise device that provides outputs to a remote display. US2008/0053452 describes apparatus for randomly varying breathing resistance and providing outputs and warnings.

Presently available apparatus suffers from various problems such as being difficult to use by patients with weak hands. Another major problem is that users often fail to use conventional apparatus regularly for the correct period and so achieve little or no benefit.

It is an object of the present invention to provide alternative respiratory apparatus.

According to the present invention, as claimed in claim 1, there is provided respiratory therapy apparatus of the above-specified kind, characterised in that the apparatus is of a generally looped shape including a first arm and a second arm attached at one end with the first arm and extending alongside the first arm and spaced from it to provide an open loop by which the apparatus can be supported, that the mouthpiece extends generally in a direction opposite from both arms from a position between both arms, that the first arm contains the first air opening and the restriction to flow of air, and that the second arm contains a second air opening and the means to record and indicate use of the apparatus, separate from the first air opening and the restriction to flow.

The apparatus preferably includes means by which the record of use of the apparatus can be downloaded to a remote computer. The means by which the record of use can be downloaded is preferably selected from one or more of the following: an electrical connector port, an infra-red transmitter and a radio transmitter. The air opening is preferably an inlet such that the patient can inhale air from the opening via the restriction. The restriction may include a valve with a spring urging a valve member against a valve seat. The force with which the valve member is urged against the valve seat may be adjustable by rotating a part of the valve. The apparatus is preferably arranged to indicate the duration of the prescribed therapy period, such as by visual means that changes between an alternating and a steady state. The visual means may be arranged to alternate during the prescribed therapy period and to change to a steady state when the prescribed therapy period has ended. The visual means preferably includes a lamp located within a translucent part of the housing of the apparatus so that the translucent part is illuminated by the lamp. The steady state is preferably an illuminated state of a predetermined duration. The apparatus preferably includes a display for representing a count of the number of breaths taken during the therapy period, the display preferably being positioned such that it is not visible by the patient during the therapy period.

Respiratory apparatus according to the present invention will now be described, by way of example, with reference to the accompanying drawings, in which:
- Figure 1: is a side elevation view of the apparatus;
- Figure 2: is a perspective view of the underside of the apparatus;
- Figure 3: is a perspective view of the apparatus resting on a flat surface;
- Figure 4: is a perspective view of the apparatus showing the saliva trap removed from the apparatus;
- Figure 5: is a perspective view of the saliva trap;
- Figure 6: is an exploded view of the apparatus showing most of its components; and
- Figure 7: is an exploded view of an inhalation valve used in the apparatus.

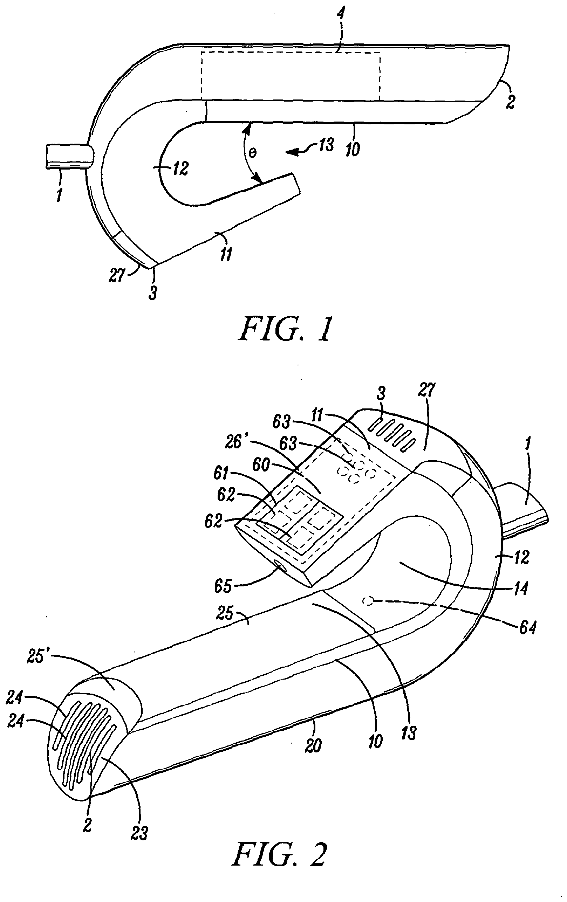

With reference first to Figures 1, 2 and 3, the apparatus has a mouthpiece 1, an air inlet 2, an air outlet 3 and a one-way inspiratory valve assembly 4 between the inlet and the mouthpiece. The valve assembly 4 provides a resistance to flow so that the user has to exert force to inhale air via the mouthpiece 1 before expelling the air through the air outlet 3. In, this way, the user can exercise his inspiratory lung muscles.

It can be seen that the apparatus has a generally looped or P shape with the air inlet 2 being located in an upper, inlet arm or handle portion 10 and the air outlet 3 being located in a lower, electronics housing 11. The lower housing 11 is connected with the handle portion 10 by a curved loop portion 12 so that the lower housing extends along and towards the handle portion at an angle θ of about 20°. The handle portion 10 is typically about 35mm wide, the lower housing 11 terminating about half way along the length of the upper portion and being spaced from it by a gap 13, about 30mm wide, to form an open loop 14.

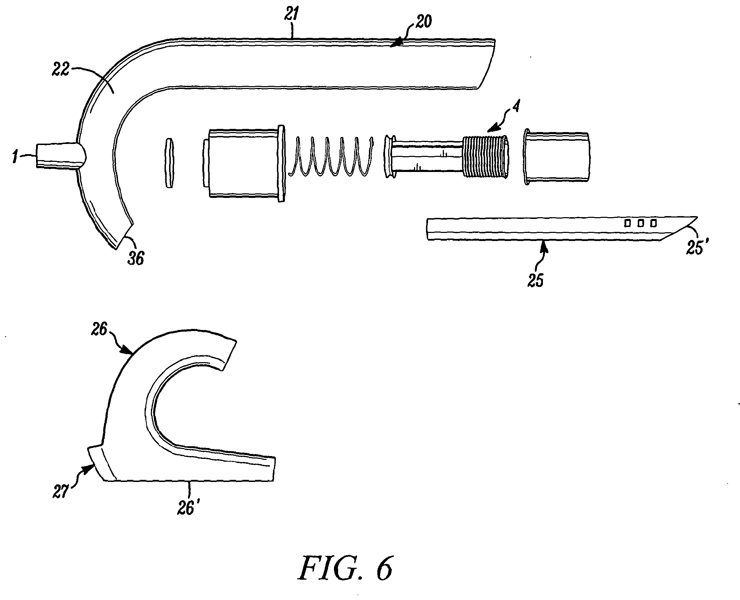

With reference now also to Figure 6, the apparatus is made of several plastics mouldings, such as of polycarbonate, clipped together in a gas-tight manner. The handle portion 10 is provided by a first transparent moulding 20 having a straight portion 21 with a curved section 22 extending around about 270°. The curved section 22 includes the mouthpiece 1 integrally formed with it and projecting rearwardly. The mouthpiece 1 tapers slightly at an angle of 4.3° and its width is about two thirds the width of the apparatus. This ensures that, if the apparatus is laid on a surface on its side, the mouthpiece 1 does not contact the surface, thereby preventing contamination. As can be seen in Figure 2, the forward end 23 of the first moulding 20 is closed apart from several parallel slots 24, which provide the air inlet 2 of the apparatus. The handle portion 10 is completed by a second moulding 25, of an opaque polycarbonate, which is straight and substantially flat. The second moulding 25 is clipped to the underside of the straight portion 21 of the first moulding 20 and can be slid forwardly along the first moulding to enable access to the inspiratory valve assembly 4.

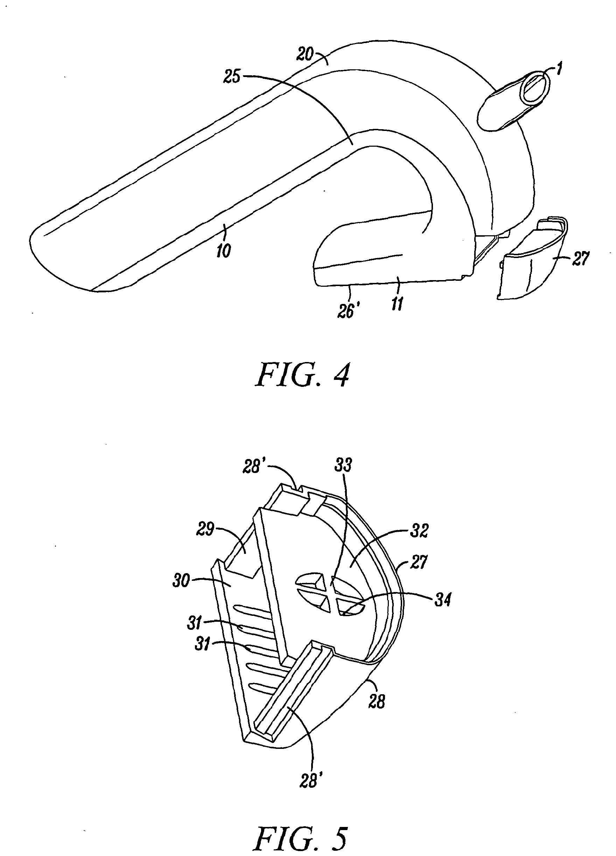

A third moulding 26 has a generally U shape providing the lower, electronics housing 11 and containing the electronics and display (to be described later) of the apparatus. The moulding 26 provides a flat, lower face 26' for the housing 11. The third moulding 26 clips to the inside of the curved rear end 22 of the first moulding 20 and abuts the rear end of the second moulding 25. The casing of the apparatus is completed by a fourth moulding 27 (Figures 4 and 5) in the form of a box-like structure forming a saliva trap of the apparatus. The saliva trap 27 has a curved front face 28 forming a continuation of the curved, outer surface of the first moulding 20 and an open rear face 29. The sides of the front face 28 have ribs 28' that enable the trap 27 to be slid down into place in the electronics housing 11. The trap 27 also has a flat lower face 30 forming a continuation of the lower face 26' of the housing 11, this lower face having a series of slots 31 formed in it to provide the air exit outlet 3. The trap 27 further includes an upper plate 32 with a central opening 33 supporting a one-way flap valve 34, which permits air to flow into the trap and out through the slots 31 but prevents air being drawn into the apparatus via the trap when the user inhales. The opening 33 aligns with an open rear end 36 of the first moulding 20.

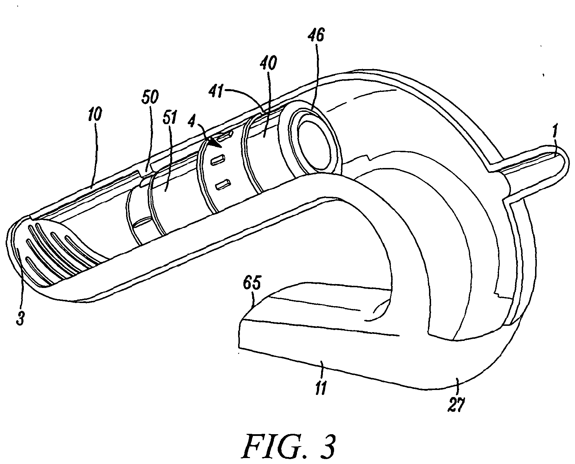

The shape of the apparatus has been chosen to make it easier to use. The flat lower face 26' of the electronics housing 11 is inclined parallel with an angled face 25' at the forward end of the second moulding 25 to enable the apparatus to be stood stably in an upright position, as shown in Figure 3, resting on these two surfaces. The slanted handle 10 forms a gap under the apparatus that makes it easy to pick up intuitively. The angled orientation of the mouthpiece 1 directs it away from surfaces to avoid contaminating either the product or the surrounding environment. The forward end 23 of the first moulding 20 through which the inlet slots 24 open is inclined away from the supporting face 25' so that the slots do not make contact with the support surface. The looped shape of the apparatus enables the user to clutch it or rest it in his hand if he becomes tired or has poor dexterity, such as caused by arthritis or other ailments. The shape enables the apparatus to be held loosely in the fingers without being dropped. The hooked shape also allows the apparatus to be hooked over a rail, equipment, furniture or the like to allow the user to take a break from holding the apparatus and yet still have it readily available

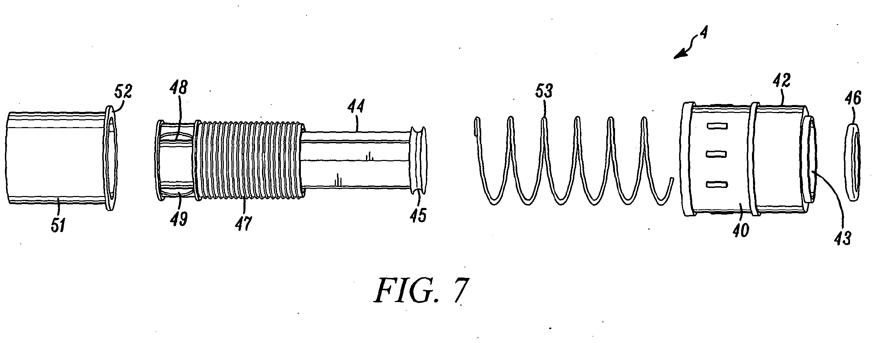

With reference now especially to Figure 7, the inspiratory valve assembly 4 is contained within the handle portion 10 trapped between the first and second mouldings 20 and 21. The valve assembly 4 includes a cylindrical valve housing 40 fixed longitudinally in position in the handle portion 10 by surface formations 41 on its inside surface. These formations 41, however, allow the valve housing 40 to be rotated. At its rear, right-hand end 42, the housing 40 has a rearwardly-facing valve seat 43. The valve assembly 4 also includes a valve member or compression column 44 extending coaxially within the housing 40 and movable along its length relative to the housing. The rear end of the column 44 is moulded with an annular groove 45 embraced by an O-ring seal 46. When the column 44 is in a forward position the seal 46 engages the seat 43 in the housing 40 to prevent air flow between the two components, and hence to prevent any air flow along the handle portion 10. The column 44 is also formed with an external screw thread 47 extending along about half of its length towards its forward end, apart from a short forward portion 48. The forward portion 48 is unthreaded but is formed with several longitudinally-extending fins 49 the purpose of which is to prevent rotation of the column 44 by engagement with corresponding formations 50 in the casing while enabling the column to slide freely along its length. A cylindrical valve platform 51 having a screw thread on its inner surface (not shown) is screwed onto the column 44. The rear end of the platform 51 has an enlarged outwardly-extending flange 52, which is engaged by the forward end of a helical spring 53. The spring 53 embraces the column 44 and is contained within the housing 40 with its rear end engaging a ledge (not shown) within the housing. The spring 53, therefore, urges the column 44 forwardly relative to the housing 40 and urges the O-ring seal 46 into sealing engagement with the valve seat 43, which limits the forward extension of the column. It can be seen that, by adjusting the position of the valve platform 51 along the column 44 the force applied by the spring 53 can be increased or decreased.

The inspiratory valve 4 is adjustable between an opening pressure difference of 5cmH₂O and 110cmH₂O in increments of 1cmH₂O. The valve pressure is set by the clinician and can be adjusted by sliding the lower moulding 25 forwardly so that the valve 4 is accessible. The clinician then rotates the valve housing 40 so as to rotate the valve platform 51 and move it longitudinally along the screw thread 47 on the valve column 44, thereby altering the compression of the spring 53. It would be preferable for the valve setting to be adjustable without having to open the apparatus so as to simplify setting and avoid exposing the clinician to any contamination within the apparatus. This could be achieved by a simple gear mechanism within the apparatus accessible by a key.

With reference to Figure 2, the electronics housing 11 contains electronics 60 and provides a display representation to the user. The flat outer, lower surface 26' of the housing 11 includes a transparent viewing screen 61 with two seven-segment LCD 62 display regions, or some other numerical display on which a count of the number of breaths taken within a defined period is represented. The main part of the housing 11 is of a translucent plastics and within this part is housed one or more lamps 63 (typically five LEDs) connected to the electronics 60. The mode of operation of the lamps 63 and numerical display 62 will be described later. The electronics unit 60 also includes some form of sensor 64 located adjacent the inspiratory valve assembly 4 to detect movement of the valve column 44. Preferably the valve column 44 incorporates a magnet and the sensor 64 in the electronics unit 60 is a Hall effect, reed switch or other form of magnet sensor. This arrangement enables the electronics housing 11 to be completely sealed. Alternative sensors could include an inductive coil to sense the presence of a ferromagnetic material, an optical sensor or the like.

The user picks up the apparatus by gripping the handle portion 10 and lifting the mouthpiece 1 up to his mouth with the viewing screen 61 lowermost. The user then places his mouth over the mouthpiece 1 and inhales. This creates a reduced pressure to the rear of the inspiratory valve 4. If the inspiratory breath was sufficiently forceful the pressure drop would be sufficient to overcome the force of the spring 53 and move the valve column 44 rearwardly a short distance, thereby lifting the seal 46 off the valve seat 43 and allowing air to flow through the valve 4 from the inlet 2. It will be appreciated that air is prevented from entering the apparatus via the outlet 3 by the one-way expiratory valve 34 in the saliva trap 27. When the user exhales, the inspiratory valve 4 closes so that the expired air flows down, opening the expiratory one-way valve 34, and out through the air outlet 3 via the saliva trap 27.

The movement of the valve column 44 is sensed by the electronics unit 60, which causes the apparatus to turn on and start a sequence of energising the LEDs 63 simultaneously to pulse on and off for the duration of the prescribed therapy period, typically of ten minutes. The LEDs 63 illuminate the interior of the electronics housing 11 and, because of its translucent nature, this causes the entire housing to pulse on and off in a manner that is visible to the user while he is exercising. The frequency of the pulsing is chosen to replicate the desired frequency of the inspiratory exercise, typically the lamps 63 glowing on for three seconds, during the inspiratory period, and then turning off for five seconds, during the expiratory period. The visual cue provided by this pulsing illumination is readily apparent to elderly users even if they are partially sighted and the pulsing illumination effect helps to steady the user's breathing rate. Audible signals, by contrast, could be more difficult especially for those who are hard of hearing. Ambient noise and the air flow noise produced by the apparatus during use could also make audible signals difficult to hear. At the end of the desired therapy period the electronics unit 60 is programmed to change the illumination effect to a steady state to indicate that the session has finished. Preferably the lamps 63 are energised to glow constantly with a mid level of illumination for a set period, typically five minutes, during which time the user can check his breath count. The user does this by turning the apparatus so that he can see the display on the screen clearly. The angle of the viewing screen 61 is chosen so that the numerical display 62 cannot be read during the therapy session but only after the user has completed his breathing exercises, removed the apparatus from his mouth and turned it over. The electronics unit 60 maintains a count of the number of breaths taken during the defined therapy period and increments the display 62 by one for each breathing cycle. The display 62 maintains the final count visible for the set period and then the apparatus powers off. Showing the breath count in this way encourages the user and builds his confidence as they see his breath count increase.

The electronics unit 60 maintains a record of operation of the apparatus, which can be downloaded to the clinician's computer by connection to a USB port 65 on the electronics housing 11. Alternatively, data could be transferred by wireless means, such as via an infra-red port or radio transmission.

The apparatus can be cleaned in various different ways, such as by autoclaving or in a domestic dishwasher. The electronics unit 60 is removed before cleaning and the inspiratory valve 4 is also removed before autoclaving. The saliva trap 27 should be removed and cleaned after each use.

The construction of the apparatus makes it particularly suitable for use with patients suffering from respiratory problems although the apparatus could also be used by athletes, divers, mountaineers, singers and wind musicians wanting to increase their inspiratory lung function. The display arrangement encourages regular use for the correct duration so as to ensure the maximum beneficial effect.

## Claims

1. Respiratory therapy apparatus including a mouthpiece (1), a first air opening (2), a restriction (4) to flow of air between the mouthpiece and the opening against which the patient breathes and means (60) to record use of the apparatus and provide an indication thereof, **characterised in that** the apparatus is of a generally looped shape including a first arm (10) and a second arm (11) attached at one end with the first arm (10) and extending alongside the first arm and spaced from it to provide an open loop by which the apparatus can be supported, that the mouthpiece (1) extends generally in a direction opposite from both arms (10 and 11) from a position between both arms, that the first arm (10) contains the first air opening (2) and the restriction (4) to flow of air, and that the second arm (11) contains a second air opening (3) and the means (60) to record and indicate use of the apparatus, separate from the first air opening (2) and the restriction (4) to flow.

2. Apparatus according to Claim 1, **characterised in that** the first and second arms (10 and 11) are arranged such that the apparatus can be supported with both arms resting on a support surface and with the mouthpiece (1) spaced above the surface.

3. Apparatus according to Claim 1 or 2, **characterised in that** the apparatus is shaped to hook over a rail or the like.

4. Apparatus according to any one of the preceding claims, **characterised in that** the apparatus includes means (60, 65) by which the record of use of the apparatus can be downloaded to a remote computer.

5. Apparatus according to Claim 4, **characterised in that** the means by which the record of use can be downloaded is selected from one or more of the following: an electrical connector port (65), an infra-red transmitter and a radio transmitter.

6. Apparatus according to any one of the preceding claims, **characterised in that** the air opening (2, 24) is an inlet such that the patient can inhale air from the opening (2, 24) via the restriction (4).

7. Apparatus according to any one of the preceding claims, **characterised in that** the restriction (4) includes a valve with a spring (53) urging a valve member (44, 45, 46) against a valve seat (43).

8. Apparatus according to Claim 7, **characterised in that** the force with which the valve member (44, 45, 46) is urged against the valve seat (43) is adjustable by rotating a part (51) of the valve.

9. Apparatus according to any one of the preceding claims, **characterised in that** the apparatus is arranged to indicate the duration of the prescribed therapy period.

10. Apparatus according to Claim 9, **characterised in that** the apparatus is arranged to indicate the duration of the prescribed therapy period by visual means (63) that changes between an alternating and a steady state.

11. Apparatus according to Claim 10, **characterised in that** the visual means (63) is arranged to alternate during the prescribed therapy period and changes to a steady state when the prescribed therapy period has ended.

12. Apparatus according to Claim 10 or 11, **characterised in that** the visual means includes a lamp (63) located within a translucent part (11) of the housing of the apparatus so that the translucent part is illuminated by the lamp.

13. Apparatus according to any one of Claims 10 to 12, **characterised in that** the steady state is an illuminated state of a predetermined duration.

14. Apparatus according to any one of the preceding claims, **characterised in that** the apparatus includes a display (62) for representing a count of the number of breaths taken during the therapy period.

15. Apparatus according to Claim 14, **characterised in that** the display (62) of count is positioned such that it is not visible by the patient during the therapy period.

## Patentansprüche

1. Atemtherapievorrichtung mit einem Mundstück (1), einer ersten Luftöffnung (2), einer Drosselung (4) für die Luftströmung zwischen dem Mundstück und der Öffnung, gegen die der Patient atmet, und einem Mittel (60) zum Aufzeichnen der Verwendung der Vorrichtung und zum Liefern einer Angabe davon, **dadurch gekennzeichnet, dass** die Vorrichtung eine im Allgemeinen schleifenförmige Gestalt mit einem ersten Arm (10) und einem zweiten Arm (11) aufweist, der an einem Ende am ersten Arm (10) befestigt und sich längs des ersten Arms erstreckt und von diesem beabstandet ist, um eine offene Schleife bereitzustellen, durch die die Vorrichtung abgestützt werden kann, dass sich das Mundstück (1) im Allgemeinen in einer Richtung entgegengesetzt zu beiden Armen (10 und 11) von einer Position zwischen beiden Armen erstreckt, dass der erste Arm (10) die erste Luftöffnung (2) und die Drosselung (4) für die Luftströmung enthält, und dass der zweite Arm (11) eine zweite Luftöffnung (3) und das Mittel (60) zum Aufzeichnen und Angeben der Verwendung der Vorrichtung separat von der ersten Luftöffnung (2) und der Drosselung (4) für die Strömung enthält.

2. Vorrichtung nach Anspruch 1, **dadurch gekennzeichnet, dass** der erste und der zweite Arm (10 und 11) derart angeordnet sind, dass die Vorrichtung abgestützt werden kann, wobei beide Arme auf einer Stützoberfläche aufliegen und wobei das Mundstück (1) über der Oberfläche beabstandet ist.

3. Vorrichtung nach Anspruch 1 oder 2, **dadurch gekennzeichnet, dass** die Vorrichtung geformt ist, um sie über einer Schiene oder dergleichen einzuhaken.

4. Vorrichtung nach einem der vorangehenden Ansprüche, **dadurch gekennzeichnet, dass** die Vorrichtung ein Mittel (60, 65) umfasst, durch das der Datensatz der Verwendung der Vorrichtung auf einen entfernten Computer heruntergeladen werden kann.

5. Vorrichtung nach Anspruch 4, **dadurch gekennzeichnet, dass** das Mittel, durch das der Datensatz der Verwendung heruntergeladen werden kann, aus einem oder mehreren der folgenden ausgewählt ist: einem elektrischen Verbindungsanschluss (65), einem Infrarotsender und einem Funksender.

6. Vorrichtung nach einem der vorangehenden Ansprüche, **dadurch gekennzeichnet, dass** die Luftöffnung (2, 24) ein Einlass ist, so dass der Patient Luft von der Öffnung (2, 24) über die Drosselung (4) einatmen kann.

7. Vorrichtung nach einem der vorangehenden Ansprüche, **dadurch gekennzeichnet, dass** die Drosselung (4) ein Ventil mit einer Feder (53) umfasst, die ein Ventilelement (44, 45, 46) gegen einen Ventilsitz (43) drückt.

8. Vorrichtung nach Anspruch 7, **dadurch gekennzeichnet, dass** die Kraft, mit der das Ventilelement (44, 45, 46) gegen den Ventilsitz (43) gedrückt wird, durch Drehen eines Teils (51) des Ventils einstellbar ist.

9. Vorrichtung nach einem der vorangehenden Ansprüche, **dadurch gekennzeichnet, dass** die Vorrichtung angeordnet ist, um die Dauer der verordneten Therapieperiode anzugeben.

10. Vorrichtung nach Anspruch 9, **dadurch gekennzeichnet, dass** die Vorrichtung angeordnet ist, um die Dauer der verordneten Therapieperiode durch ein visuelles Mittel (63) anzugeben, das sich zwischen einem abwechselnden und einem stetigen Zustand ändert.

11. Vorrichtung nach Anspruch 10, **dadurch gekennzeichnet, dass** das visuelle Mittel (63) angeordnet ist, um während der verordneten Therapieperiode abzuwechseln, und sich auf einen stetigen Zustand ändert, wenn die verordnete Therapieperiode geendet hat.

12. Vorrichtung nach Anspruch 10 oder 11, **dadurch gekennzeichnet, dass** das visuelle Mittel eine Lampe (63) umfasst, die innerhalb eines durchsichtigen Teils (11) des Gehäuses der Vorrichtung angeordnet ist, so dass der durchsichtige Teil durch die Lampe beleuchtet wird.

13. Vorrichtung nach einem der Ansprüche 10 bis 12, **dadurch gekennzeichnet, dass** der stetige Zustand ein beleuchteter Zustand einer vorbestimmten Dauer ist.

14. Vorrichtung nach einem der vorangehenden Ansprüche, **dadurch gekennzeichnet, dass** die Vorrichtung eine Anzeige (62) zum Darstellen eines Zählwerts der Anzahl von während der Therapieperiode durchgeführten Atemzügen umfasst.

15. Vorrichtung nach Anspruch 14, **dadurch gekennzeichnet, dass** die Anzeige (62) des Zählwerts derart positioniert ist, dass sie für den Patienten während der Therapieperiode nicht sichtbar ist.

## Revendications

1. Appareil de thérapie respiratoire, comportant un embout (1), une première ouverture d'air (2), une restriction (4) du flux d'air entre l'embout et l'ouverture contre laquelle le patient respire et des moyens (60) pour enregistrer l'utilisation de l'appareil et fournir une indication correspondante, **caractérisé en ce que** l'appareil est généralement en forme de boucle incluant un premier bras (10) et un second bras (11) attachés à une extrémité au premier bras (10) et s'étendant le long du premier bras, et espacé de celui-ci pour fournir une boucle ouverte par laquelle l'appareil peut être supporté, **en ce que** l'embout (1) s'étend généralement dans une direction opposée aux deux bras (10 et 11) depuis une position située entre les deux bras, **en ce que** le premier bras (10) comprend la première ouverture d'air (2) et la restriction (4) du flux d'air, et **en ce que** le second bras (11) comprend une seconde ouverture d'air (3) et les moyens (60) pour enregistrer et indiquer l'utilisation de l'appareil, séparé de la première ouverture d'air (2) et de la restriction (4) du flux.

2. Appareil selon la revendication 1, **caractérisé en ce que** le premier et le second bras (10 et 11) sont disposés de telle manière que l'appareil puisse être supporté avec les deux bras reposant sur une surface de support et avec l'embout (1) espacé au-dessus de la surface.

3. Appareil selon la revendication 1 ou 2, **caractérisé en ce que** l'appareil est formé pour s'accrocher sur un rail ou similaire.

4. Appareil selon l'une quelconque des revendications précédentes, **caractérisé en ce que** l'appareil comprend des moyens (60, 65) par lesquels l'enregistrement de l'utilisation de l'appareil peut être téléchargé sur un ordinateur distant.

5. Appareil selon la revendication 4, **caractérisé en ce que** les moyens par lesquels l'enregistrement de l'utilisation pouvant être téléchargé, sont sélectionnés parmi un ou plusieurs des éléments suivants : un port de connecteur électrique (65), un transmetteur infrarouge et un transmetteur radio.

6. Appareil selon l'une quelconque des revendications précédentes, **caractérisé en ce que** l'ouverture d'air (2, 24) est une entrée telle que le patient peut inhaler l'air de l'ouverture (2, 24) via la restriction (4).

7. Appareil selon l'une quelconque des revendications précédentes, **caractérisé en ce que** la restriction (4) comprend une soupape avec un ressort (53) poussant un organe de soupape (44, 45, 46) contre un siège de soupape (43).

8. Appareil selon la revendication 7, **caractérisé en ce que** la force avec laquelle l'organe de soupape (44, 45, 46) est poussé contre le siège de soupape (43) est ajustable en faisant tourner une partie (51) de la soupape.

9. Appareil selon l'une quelconque des revendications précédentes, **caractérisé en ce que** l'appareil est agencé pour indiquer la durée de la période de thérapie prescrite.

10. Appareil selon la revendication 9, **caractérisé en ce que** l'appareil est agencé pour indiquer la durée de la période de thérapie prescrite par des moyens visuels (63) qui changent entre un état alternatif et un état stable.

11. Appareil selon la revendication 10, **caractérisé en ce que** les moyens visuels (63) sont agencés pour alterner pendant la période de thérapie prescrite et pour changer en un état stable lorsque la période de thérapie prescrite est terminée.

12. Appareil selon la revendication 10 ou 11, **caractérisé en ce que** les moyens visuels incluent une lampe (63) disposée dans une partie translucide (11) de l'habitacle de l'appareil, de sorte que la partie translucide est illuminée par la lampe.

13. Appareil selon l'une quelconque des revendications 10 à 12, **caractérisé en ce que** l'état stable est un état illuminé d'une durée prédéterminée.

14. Appareil selon l'une quelconque des revendications précédentes, **caractérisé en ce que** l'appareil comprend un écran (62) pour représenter un décompte du nombre de respirations prises pendant la période de thérapie.

15. Appareil selon la revendication 14, **caractérisé en ce que** l'écran (62) de décompte est positionné de sorte qu'il n'est pas visible du patient pendant la période de thérapie.
